# EUROPEAN PATENT APPLICATION

(11) **EP 4 230 117 A1**
(43) Date of publication of application: **23.08.2023**
(21) Application number: 22157421.3
(22) Date of filing: 18.02.2022
(51) Int. Cl.: A61B 5/00, A61B 5/026, A61B 5/0295

(54) **A SYSTEM AND METHOD FOR TISSUE ANALYSIS USING REMOTE PPG**

(71) Applicant: Koninklijke Philips N.V., 5656 AG Eindhoven (NL)
(72) Inventor: LAI, Marco, Eindhoven (NL); HENDRIKS, Bernardus Hendrikus Wilhelmus, Eindhoven (NL); LUCASSEN, Gerhardus Wilhelmus, Eindhoven (NL); NOTTEN, Marc Godfriedus Marie, Eindhoven (NL); DE WILD, Nico Maris Adriaan, Eindhoven (NL); VERBAKEL, Frank, Eindhoven (NL); SCHULEPOV, Sergei Y., Eindhoven (NL)
(74) Representative: Philips Intellectual Property & Standards

(57) **Abstract**

A system and method for PPG analysis processes images to determine PPG signals from different image regions of the images and determine relative delays between the PPG signals for the different image regions. The time alignment between the PPG signals is improved so that a more accurate global PPG signal is obtained for the overall region of interest. In this way, PPG signals are realigned or partially realigned to provide an overall PPG signal with improved signal to noise ratio.

## Description

### FIELD OF THE INVENTION

This invention relates to the analysis of tissue using photoplethysmography (PPG) analysis.

### BACKGROUND OF THE INVENTION

Microcirculation is the circulation of the blood in the smallest blood vessels, present in the vasculature embedded within organ tissues. The structure of the microvasculature is extremely complex, as so many capillaries are present in the tissues, with the individual capillaries so convoluted that the blood flow at any given point in any given capillary could be in any direction. For this reason, it can be stated that their overall function becomes averaged. That is, there is an average rate of blood flow through each tissue capillary bed, an average capillary pressure within the capillaries, and an average rate of transfer of substances between the blood of the capillaries and the surrounding interstitial fluid. This is the perfusion of the tissue.

Organ perfusion is a crucial indicator of injury and disease, which may include inflammation, stagnant or stopped blood flow, and all pathologies that can lead to global tissue hypoxia and organ dysfunction. Perfusion monitoring can be used to assess these microvascular injuries and many others, such as the progress of healing of either burned skin or wounds or the recovery of the perfusion downstream of a vessel lesion, and the necrosis (e.g., foot ulceration, sepsis) for patients with diabetes.

Non-contact PPG imaging is a recent emerging technology able of monitoring skin perfusion. PPG imaging utilizes an off-the-shelf camera and a light source to remotely detect the dynamic changes in blood volume beneath the skin and allows extracting blood pulsation signals. PPG imaging allows for the elaboration of large tissue areas, thus building a so-called perfusion map, which is a great advantage with respect to contact PPG. PPG imaging has shown to be capable of detecting skin perfusion perturbations, such as irritation, temperature changes and even flow blockage during pressure measurements and has even been evaluated for peripheral arterial disease assessment.

It is possible to build an amplitude map, that represents the amplitude of the PPG signal per image sensing pixel. Furthermore, it is also possible to build a delay map, which provides a measure of the average time delay between the PPG signal wave of each pixel and a reference PPG signal. There is a small delay in the blood pulsation arrival, due to small differences in the microcirculatory bad, such as the resistance and elasticity of the vessels, as well as different artery branches that supply the recorded tissues.

For the purposes of this application, which relates to processing of non-contact PPG signals, perfusion may be defined as the amplitude of a PPG signal of the tissue, namely the amplitude of the pulsatility extracted from the tissue.

It is well known that the perfusion level is not the same everywhere in the body. For example, in a study by the applicant, PPG imaging of the hands and feet were studied, and it was found that the perfusion level is higher on the palms of the hands rather than on the sole of the feet. This is also true for organs, such as the intestine. The small intestine and the colon have different functions, are far away and are supplied by different artery branches, and accordingly have different perfusion levels.

If different tissue types are present in a single image of a remote PPG camera, a general PPG image analysis will average these differences. For example, if an anastomosis is being performed (e.g. after removal of part of the intestine) and the level of perfusion is measured via PPG imaging, there are different intestines in the image. The image analysis will also not show whether the perfusion difference is physiological or due to a lesion (e.g. ischemia, inflammation).

For example, during open bowel resection, a part of the colon or small intestine is surgically removed to remove a tumor. First, a surgeon mobilizes the bowel from the surrounding organs and membranes. The bowel is clamped on both sides of the tumor and blood flow towards that part is interrupted. The surgeon will then cut out the diseased part of the bowel and stitch the two clamped sides back together, which is called the anastomosis. The intestinal anastomosis is to establish communication between two formerly distant portions of the intestine. This procedure restores intestinal continuity after removal of the pathological condition affecting the bowel. If the anastomosis does not properly heal due to inadequate perfusion, leakages occur. Leakages are dangerous and can lead to severe infections and require hospitalization and reoperation. These leakages occur in 5 to 10% of patients undergoing bowel resection. Here, objective perfusion parameters could help identify inadequate healing.

A PPG signal for an area of interest is obtained by averaging the values of all the pixels for that area for each frame. The resulting average PPG signal can be used to assess the heart rate of the patient, as well as a perfusion level.

For enhancing the strength of the average PPG signal, weighting average methods have been proposed, where the PPG signal of each pixel is weighted with the signal-to-noise ratio (SNR) of the PPG signal. The higher the SNR of the PPG signal of that pixel, the more that pixel contributes to the weighted average.

However, if a large area of the skin or an organ is recorded, or if far apart areas are recorded simultaneously with a camera, the delay in PPG time arrival influences the average PPG signal. Thus, taking a global average in this case degrades the signal to noise ratio of the signal.

### SUMMARY OF THE INVENTION

The invention is defined by the claims.

According to examples in accordance with an aspect of the invention, there is provided a system for PPG analysis, comprising a processor adapted to receive images of a region of interest and to process the images to:
determine PPG signals from different image regions of the images;
determine relative delays between the PPG signals for the different image regions;
improve the alignment between the PPG signals in time; and
determine a global PPG signal for the overall region of interest by combining the time-adapted PPG signals.

By determining the different delays for different image regions (i.e. image pixels corresponding to image sensor pixels), it is possible to realign or partially realign the PPG signals so that a global signal for the overall region of interest may be obtained with improved signal to noise ratio. The destructive interference caused by averaging PPG signals with different time delays is thereby reduced or avoided.

The PPG signals may be aligned with corresponding timing (i.e. the PPG signals are perfectly synchronized to a reference signal). However, as long as the delay between the local PPG signal and a reference PPG signal decreases, the destructive interference caused by averaging PPG signals with different time delays is reduced, and therefore an improvement of the global PPG signal is obtained.

By way of example, the average timing difference compared to a reference PPG signal is reduced to below 50% of its initial value, or below 40%, or below 30%, or below 20%.

In the case of an anastomosis, by averaging the signals from the two different tissue types, e.g. the two ends of the intestine, a smoother PPG signal is found.

The processor is for example adapted to:
derive a reference PPG signal;
determine the relative delays between the PPG signals by determining a delay of each PPG signal relative to the reference PPG signal; and
align the PPG signals in time by providing a common delay relative to the reference PPG signal.

Thus, in this case, the PPG signals are time-aligned relative to a reference and hence relative to each other.

The reference PPG signal for example comprises:
an average PPG signal for the region of interest; or
an average PPG signal for a portion of the region of interest;
a PPG signal for an individual image region; or
a PPG signal from an area of the body outside the region of interest.

The reference PPG signal is in all cases acquired simultaneously with the image sequence of the region of interest.

The relative delays for example comprise a delay relative to a reference PPG signal. The delay is computed as the average time delay between the PPG signal wave of each pixel and the reference PPG signal, over a certain time period.

The length of the time period includes at least a heartbeat cycle and the reference PPG signal is obtained as a spatial average of the all pixel values of a video (or series of images) which is a time-dependent signal modulated at the heart rate. A signal is created which varies over time, frame by frame.

The processor is for example adapted to generate and output a PPG perfusion map.

The remote PPG sensing is thus used to derive a PPG perfusion map from PPG amplitude levels. Remote PPG sensing can thus show perfusion levels at different locations as well as providing an accurate overall perfusion level.

The processor is for example adapted to generate and output a PPG delay map.

The PPG delay map shows relative delays of arrival of the pulsatile blood flow to the tissue. The delay information is for different image regions, i.e. the image data associated with different pixels of the image sensor.

The processor is for example adapted to determine a global PPG signal for the overall region of interest by averaging the aligned PPG signals.

The system may comprise a camera for capturing the images of the region of interest.

The camera may comprise:
a 2D camera or a set of 2D cameras; or
a hyperspectral camera or a set of hyperspectral cameras.

The invention also provides a computer-implemented method for processing images to derive remote PPG signals, comprising:
receiving images of a region of interest;
determining PPG signals from different image regions of the images;
determining relative delays between the PPG signals for the different image regions;
improving the alignment between the PPG signals in time; and
determining a global PPG signal for the overall region of interest by combining the time-adapted PPG signals.

The method may comprise:
deriving a reference PPG signal;
determining the relative delays between the PPG signals by determining a delay of each PPG signal relative to the reference PPG signal; and
aligning the PPG signals in time by providing a common delay relative to the reference PPG signal.

The reference PPG signal may be obtained by:
averaging the PPG signals for the region of interest; or
averaging the PPG signals for a portion of the region of interest; or
obtaining a PPG signal for an individual image region.

The method may comprise:
deriving a PPG perfusion map based on PPG amplitude levels at the image regions; and/or
deriving a PPG delay map based on PPG relative delays between the different image regions.

The invention also provides a computer program comprising computer program code means which is adapted, when said program is run on a computer, to implement the method defined above. The invention also provides a processor which is programed with the computer program.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a representation of a frame of a video acquired of the intestine and a global PPG signal derived from the video;
Figure 2 shows separate PPG signals for separate regions of interest;
Figure 3 shows a PPG perfusion map;
Figure 4 shows a zoom-in of the PPG signals extracted from the two regions of interest from Figure 2 and a PPG delay map;
Figure 5 shows an image of an intestinal area after open bowl resection, the corresponding amplitude map and the corresponding delay map;
Figure 6 an average PPG amplitude for each area;
Figure 7 shows a plot of a reference PPG signal and a plot of a PPG signal from one pixel of the camera image;
Figure 8 shows the same plot of a reference PPG signal and a plot of the same PPG pixel signal but synchronized with the reference PPG signal;
Figure 9 shows the effect on the delay map of the pixel synchronization;
Figure 10 shows a comparison of the average PPG signal obtained by simply averaging all the PPG signals and the average PPG signal after the PPG signals are synchronized;
Figure 11 shows the frequency spectra for the simply averaged PPG signal;
Figure 12 shows the frequency spectra for the synchronized average PPG signal;
Figure 13 shows system for tissue analysis;
Figure 14 shows a method for tissue analysis; and
Figure 15 shows a hypercube of a hyperspectral camera;

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for PPG analysis which processes images to determine PPG signals from different image regions of the images and determine relative delays between the PPG signals for the different image regions. The time alignment between the PPG signals is improved so that a more accurate global PPG signal is obtained for the overall region of interest. In this way, PPG signals are realigned or partially realigned to provide an overall PPG signal with improved signal to noise ratio.

Before describing the system and method of the invention, the known operation of a remote PPG imaging system, and the known image processing methods, will first be described.

Remote PPG imaging enables a determination of tissue perfusion from images captured of the tissue of interest, e.g. the skin or even tissue beneath the skin. Remote PPG typically uses ambient light, functioning as broad band white light source, and the diffuse and specular reflections are analyzed for different color components. Remote PPG imaging may be used to construct a PPG amplitude map and a PPG delay map.

For this purpose, a camera or a series of cameras (at one or multiple wavelengths) captures video of the tissue area, e.g. skin, at a distance. The measurements derived from the video are remote PPG images, which provide non-contact measurement of a pulse signal by analyzing subtle changes of skin color (or organ color) i.e. at different wavelengths of the light.

It has been proposed to use remote PPG for inflammation detection. It has also been proposed in European Patent Application No. 20214955.5 to measure perfusion based both on a PPG amplitude level and also information about the distribution of the PPG phases, such as a standard deviation or interquartile range of a phase map.

By extracting pulse signals at each individual location of the skin region (corresponding to each pixel of the cameras) a spatial map of the pulse signal can be derived, showing both amplitude and delay. This perfusion map thus represents the amplitude and delay of the PPG signal per pixel and hence per location of the skin.

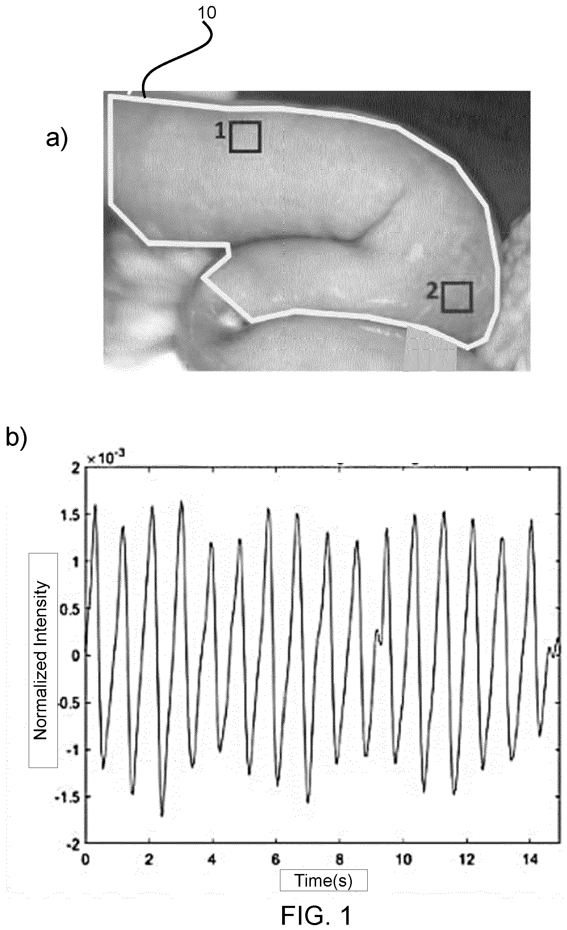

Figure 1 (a) shows a representation of a frame of a video acquired of the intestine. By spatial averaging the pixel values in the region of interest (ROI) 10, a signal can be derived from the video, modulated at the heart rate. This signal is shown in Figure 1 (b) as a normalized intensity of the PPG signal versus time.

The PPG signal of Figure 1 (b) represents an average for the whole region of interest.

However, separate signals may also be captured from smaller regions of interest, shown as region 1 and region 2 in Figure 1 (a).

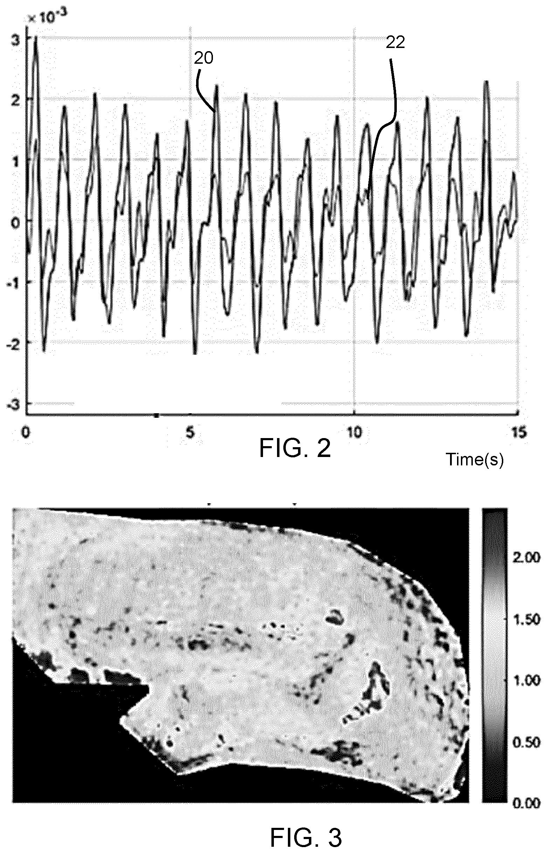

Figure 2 shows separate PPG signals, as a first signal 20 for the first region of interest and a second signal 22 for the second region of interest. The two PPG signals are modulated at the same heart rate frequency, but show a different amplitude. By extracting the amplitude from the PPG signal of each separate region of tissue, i.e. from each corresponding pixel of the captured video, a PPG perfusion map may be obtained, as shown in Figure 3.

Figure 3 is a black and white representation. However, the PPG perfusion may be color-coded, for example assigning a more red color to areas with higher perfusion and a more blue color to areas with lower perfusion. The PPG amplitude map thus represents the amplitude of the PPG signal per pixel and hence per location of the skin or other tissue being analyzed.

Additional valuable information may be obtained by analyzing the PPG signals.

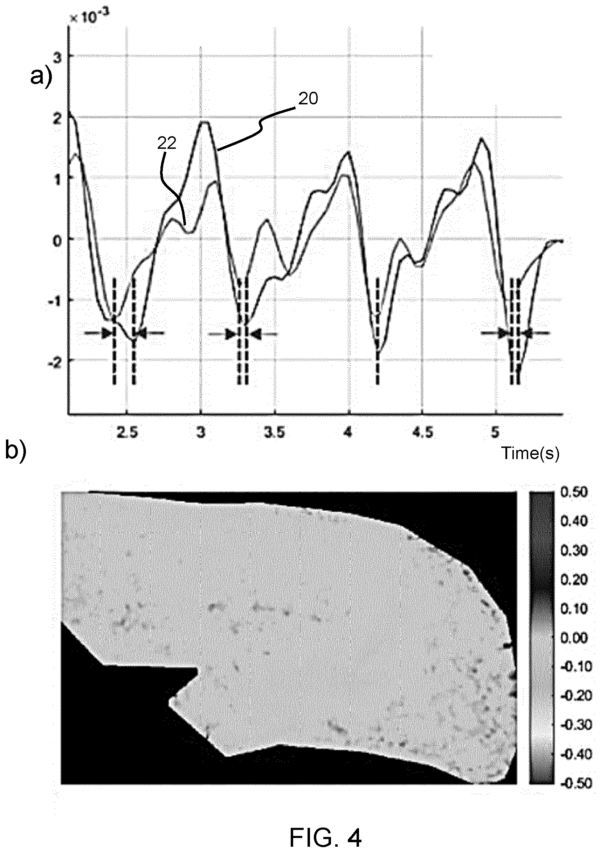

Figure 4 (a) shows a zoom-in of the PPG signals extracted from the two regions of interest from Figure 2. Even though the signals are extracted simultaneously from the same organ, the pulsation arrives slightly before in one area than in the other. There is a small delay in the blood pulsation arrival, due to small differences in the microcirculatory bed, such as the resistance and elasticity of the vessels, as well as different artery branches that supply the recorded tissues.

The delays of the PPG signals of each pixel with respect to a reference signal may be extracted and used for building a delay map. The delay map is shown in Figure 4.

Since the delay between the signals is not always constant but varies during the acquisition, an average delay is used between signals per pixel.

The average delay represents the average time delay between the PPG signal of each pixel and a reference PPG signal. In this way a signal in time is built. The length of the PPG signal should include at least a heartbeat cycle, so it is subject dependent.

The average delay is a value of delay assigned to each pixel. From the plot of Figure 4, it can be seen that there is a difference in time arrival between the peaks of the PPG signals. Therefore, for each pixel, the average of these delay (with respect to a reference signal) is assigned. At the end, the delay map is built, where each pixel contains the average delay between the PPG signal of that pixel and the reference PPG signal.

By acquiring a video stream, a series of images is obtained over time, for example with a frame rate of 20 frames per second. In order to compute the global PPG signal over time, the pixel values of the frame 1 are spatially averaged, so that the 2D set of pixels yields one value. The pixels of frame 2 are then averaged, and so on.

Eventually, a PPG signal is obtained over time (with the same length as the video that has been acquired), where each value of the signal is a spatial average of one corresponding frame of the video acquired. The image frames for example comprise 968x728 pixels, by way of example.

The PPG signal of each pixel is thus compared with the global PPG signal being used as a reference. The value assigned to each pixel "n" of the delay map thus represents the average delay between the PPG signal in the pixel "n" and the global PPG signal being used as a reference. Thus, the value of the delay for each pixel in the PPG delay map represents the average delay (in terms of average time shift) between the PPG signal of that pixel and the global PPG signal. The delays are for example computed by using the lock-in amplification algorithm.

The delay map thereby provides a measure of the average time delay between the PPG signal wave of each pixel and the reference PPG signal for at least one heartbeat cycle. The reference PPG signal is the global PPG signal of Figure 1 (b) extracted from the entire region on interest of the intestine.

Since the reference signal is extracted from the entire region of interest, a PPG signal from a given location of the image is likely to be in phase with the reference.

Similar to the map of the amplitude, Figure 4 (b) is a black and white representation. However, the PPG delay map may be color-coded. The Hue, Saturation, Value (HSV) color system is for example employed, since it works well with the periodicity of the PPG signal.

For extracting the amplitude maps and delay maps, a lock-in amplification method may be used.

Details on how to calculate the PPG maps using the lock-in amplification method can be found in:
(i) Lai M, Shan C, Ciuhu-Pijlman C, Izamis ML. Perfusion monitoring by contactless photoplethysmography imaging, 2019 IEEE 16th International Symposium on Biomedical Imaging (ISBI 2019) 2019 Apr 8 (pp. 1778-1782). IEEE; and
(ii) Lai M, Dicorato CS, de Wild M, Verbakel F, Shulepov S, Groen J, Notten M, Lucassen G, van Sambeek MR, Hendriks BH. Evaluation of a non-contact Photo-Plethysmographic Imaging (iPPG) system for peripheral arterial disease assessment, Medical Imaging 2021: Biomedical Applications in Molecular, Structural, and Functional Imaging 2021 Feb 15 (Vol. 11600, p. 116000F). International Society for Optics and Photonics.

Because of the great advantages that the remote PPG technology has shown for remotely and non-invasively assessing skin-level perfusion, PPG imaging can be translated to organ perfusion assessment for detecting the perfusion of the microvasculature tissue bed beneath the organ surface, without any modification to the current acquisition setup.

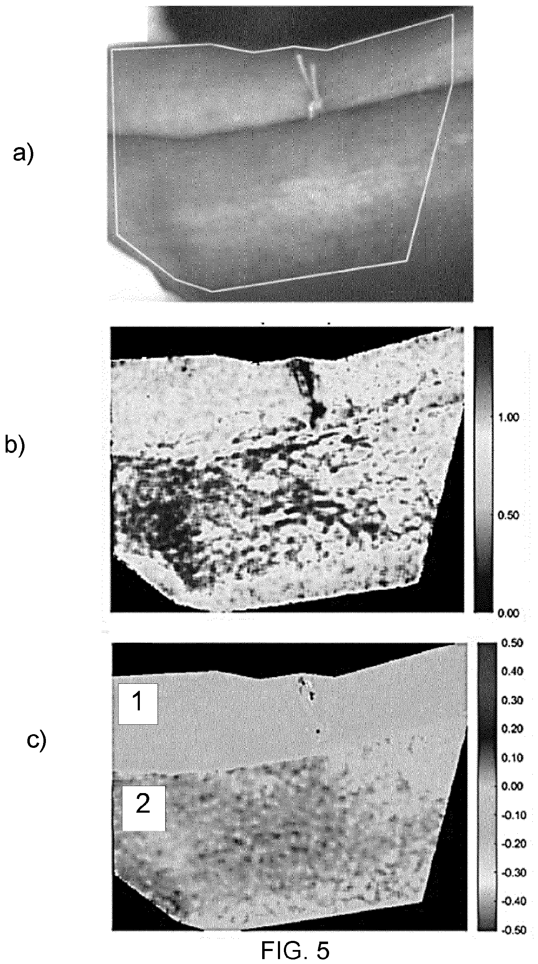

Figure 5(a) shows an image of an intestinal area after open bowl resection. Figure 5(b) shows the corresponding amplitude map (normalized with respect to the maximum value) and Figure 5(c) shows the corresponding delay map.

The delay map clearly shows that there are two areas which each have a relatively uniform delay value but they are different to each other (in color, the two images are predominantly different colors, such as blue and green).

The areas are supplied by different arterial branches and there is a relative difference in the PPG time arrival between the areas. This could be assessed by comparing the PPG signals extracted from the two areas.

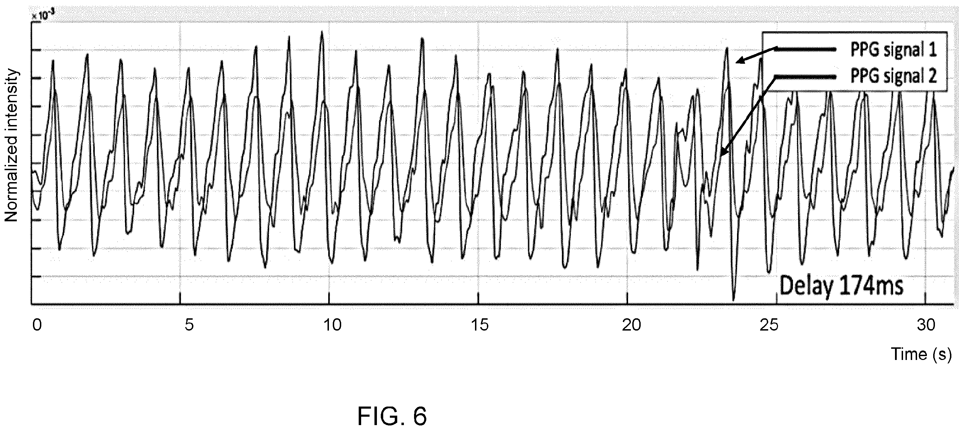

Figure 6 the average PPG amplitude for each area. The PPG amplitude level is different, but it can also be seen that one PPG signal arrives slightly before the other. The relative delay is 174ms.

In the case of Figure 1, the delay between different areas is relatively small and is mainly due to slight changes in vascular resistance and compliance. However, the delay seen in Figure 6 is dependent on the PPG arrival time in the two areas.

The two areas will each have a spread of different delay times. However each area will have a different average and an associated spread. Thus, ranges of PPG delay times, from the PPG delay map, may be used for classification.

For example, two areas of a PPG delay map may be considered separate if the difference of their delays is larger than a threshold such as 20ms, preferably more than 50ms and even more preferred larger than 100ms.

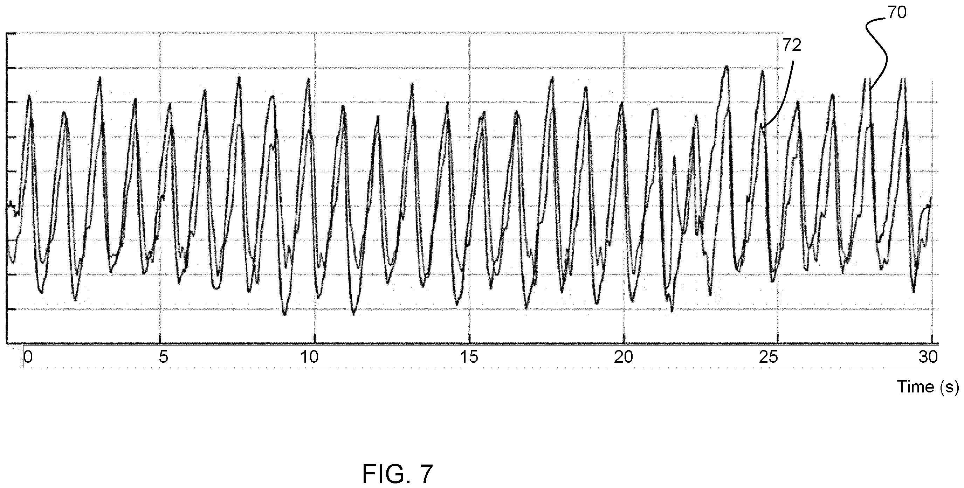

The approach of the invention is explained with referenced to Figures 7 to 11.

Figure 7 shows a plot 70 of a reference PPG signal and a plot 72 of a PPG signal from one pixel of the camera image.

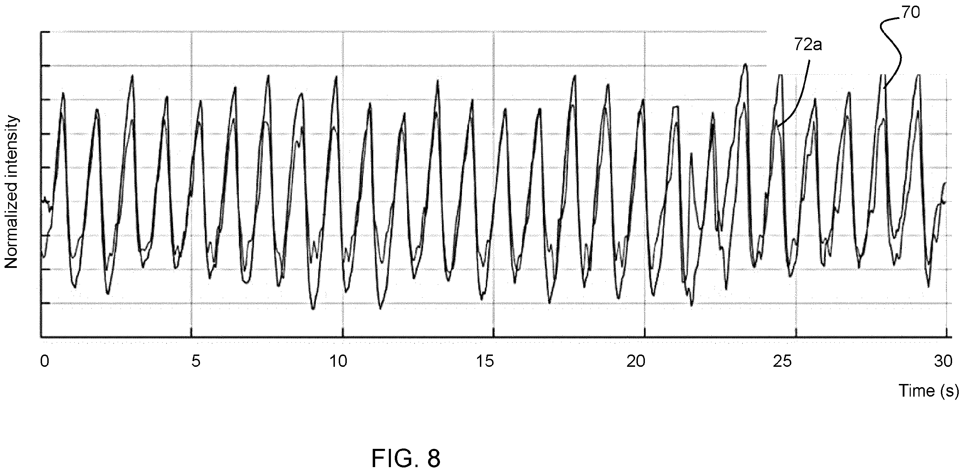

Figure 8 shows the same plot 70 of a reference PPG signal and a plot 72a of the same PPG pixel signal but synchronized with the reference PPG signal.

The reference PPG signal 70 may be computed by averaging the pixel values of the region of interest (ROI) from each frame. The reference signal could instead be the average PPG signal from a portion of the image, or the PPG signal from a single pixel, or even a PPG signal extracted from another area of the body, for example using a contact probe (using standard PPG).

The delay of the PPG signal 72 of each pixel, with respect to the reference signal, is computed so that the synchronization of the PPG signal of each pixel can take place relative to the reference signal as shown in Figure 8.

In this way, all the synchronized PPG signals of each pixels are averaged, to result in a PPG signal with higher SNR than the simple average of all the pixels PPG signals without synchronization.

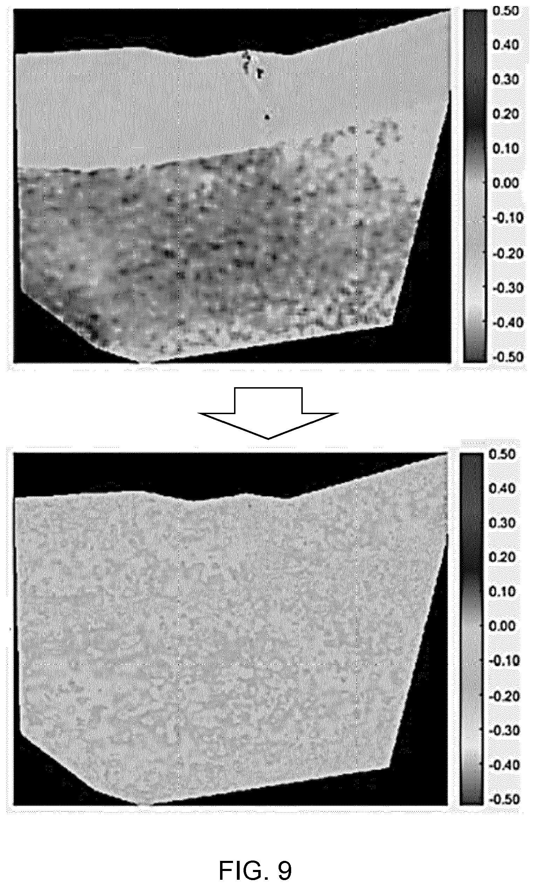

Figure 9 shows the effect on the delay map of the pixel synchronization. The top image is a delay map before PPG signal synchronization and the bottom image is a delay map after PPG signal synchronization.

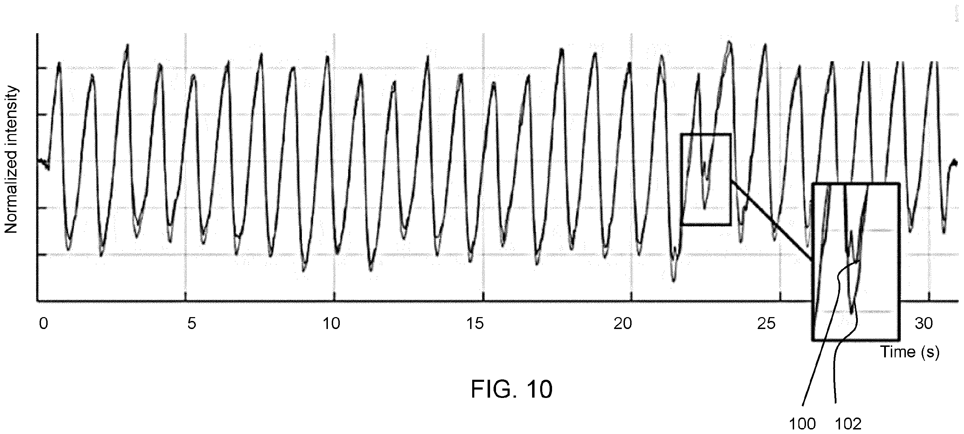

Figure 10 shows a comparison of the average PPG signal 100 obtained by simply averaging all the PPG signals (in this case the average PPG signal is also the reference signal), with respect to the average PPG signal 102 after the PPG signals are synchronized.

The enlarged area in Figure 10 shows that artifacts in the average signal are reduced.

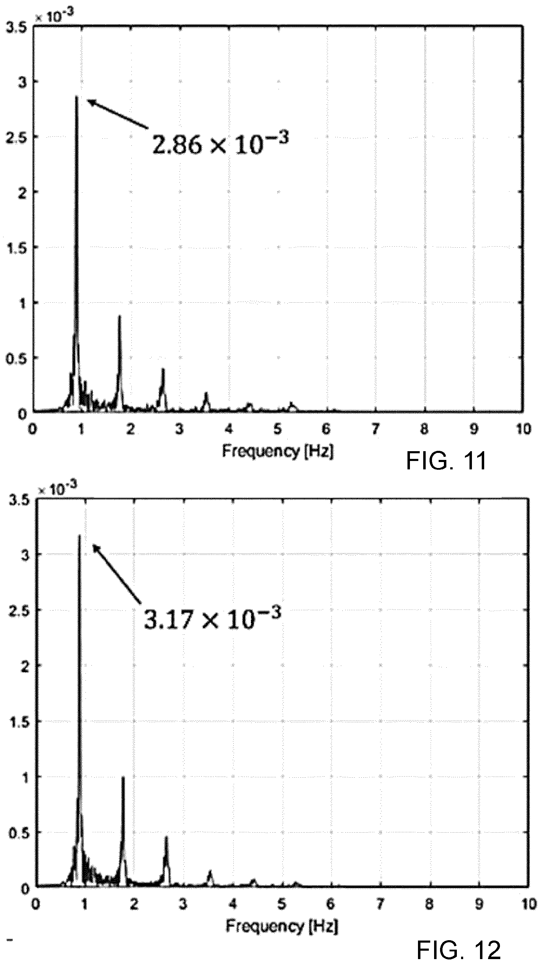

Figure 11 shows the frequency spectra for the average PPG signal 100 and Figure 12 shows the frequency spectra for the average PPG signal 102.

The spectra of the two signals show that the synchronized PPG signal peak is higher than the more simple average signal peak. Thus, the signal to noise ratio is increased. This approach enables synchronization of two or more PPG signals from different and far apart areas so that a more accurate general PPG analysis may be performed.

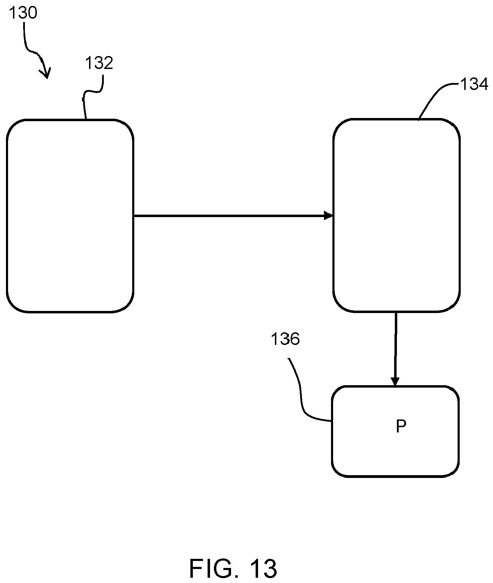

Figure 13 shows system 130 for tissue analysis. The system comprises one or more imaging cameras 132 and a processor 134 adapted to receive the images and process the images. The image processing creates a more accurate perfusion measure P.

The system has a display 136 for displaying the output, for example as a perfusion map and a delay map and/or as PPG signals over time.

The tissue classification may use machine learning algorithms, such as Support Vector Machines (SVMs) and Convolutional Neural Networks (CNNs).

The field of view of the camera could include all of the organs inside the abdomen or only a part of the organs inside. By classifying the tissue types present in the field of view, a comparison of perfusion is prevented between tissues which can have different levels of perfusion due to their different functions. These different tissues can even be supplied by different artery branches.

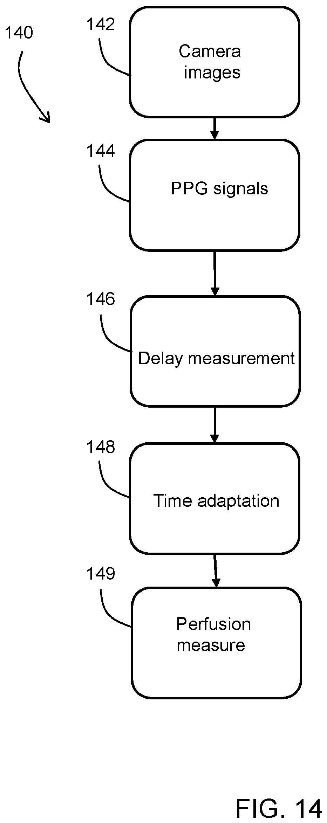

Figure 14 shows a computer-implemented tissue analysis method 140. The method 140 comprises receiving image sensor images in step 142.

PPG signals are determined in step 144 from different image regions of the images.

Relative delays between the PPG signals for the different image regions are determined in step 146, and the signals are adapted in step 148 to improve the alignment between the PPG signals in time. A global PPG signal for the overall region of interest is then obtained in step 149 by combining the time-adapted PPG signals and hence enabling a perfusion measure to be obtained.

The remote PPG sensing used in the system and method described above may be performed using broad band illumination, such as using ambient light and visible light cameras.

However, there is also the option of using hyperspectral imaging. Hyperspectral imaging (HSI) is an emerging imaging modality for medical applications that offers great potential for non-invasive disease diagnosis and surgical guidance. The objective of hyperspectral imaging is to collect a three-dimensional dataset of spatial and spectral information, known as hypercube.

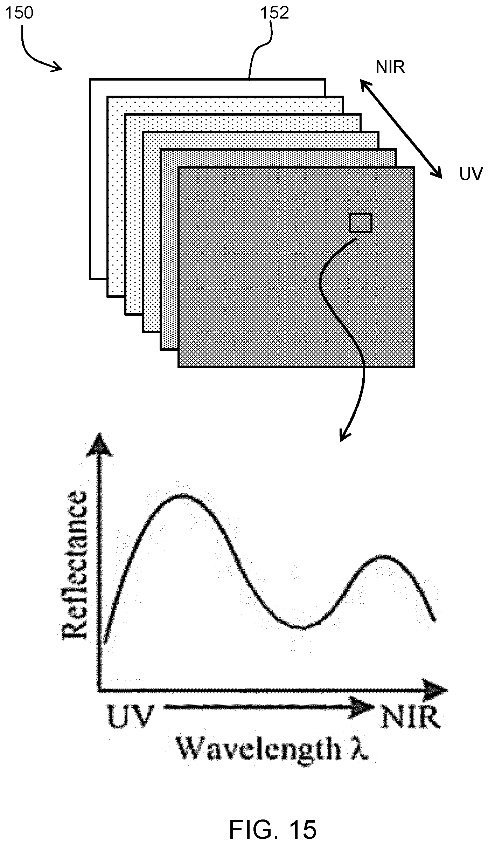

As shown in Figure 15, the hypercube is three-dimensional dataset 150 comprising two-dimensional images 152 at each of a set of wavelengths. Figure 15 also shows a reflectance curve (i.e. the spectral signature) of a pixel in each image.

The use of hyperspectral images allows additional image processing to be performed, for example it enables a level of oxygenation of tissue to be obtained. A contact PPG probe contains two LEDs and photodetectors at two different wavelengths. By combining the readings at these two wavelengths, an estimation of the oxygen level is possible. For non-contact PPG, RGB cameras have red, green, and blue channels but they are sensitive to much broader wavelength ranges, so extracting oxygenation from a normal RGB camera is not possible. The HSI camera acquires images at specific and narrow wavelengths, so the images can be combined to extract oxygenation values.

The use of hyperspectral images allows additional image processing to be performed, for example it enables a level of oxygenation of tissue to be obtained.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system for PPG analysis, comprising a processor (134) adapted to receive (142) images of a region of interest and to process the images to:
(144) determine PPG signals from different image regions of the images;
(146) determine relative delays between the PPG signals for the different image regions;
(148) improve the alignment between the PPG signals in time; and
(149) determine a global PPG signal for the overall region of interest by combining the time-adapted PPG signals.

2. The system of claim 1, wherein the processor is adapted to:
derive a reference PPG signal;
determine the relative delays between the PPG signals by determining a delay of each PPG signal relative to the reference PPG signal; and
align the PPG signals in time by providing a common delay relative to the reference PPG signal.

3. The system of claim 2, wherein the reference PPG signal comprises:
an average PPG signal for the region of interest; or
an average PPG signal for a portion of the region of interest;
a PPG signal for an individual image region; or
a PPG signal from an area of the body outside the region of interest.

4. The system of any one of claims 1 to 3, wherein said relative delays comprise a delay relative to a reference PPG signal which is an average delay time period for all image regions for a frame of image data.

5. The system of any one of claims 1 to 4, wherein the processor is adapted to generate and output a PPG perfusion map.

6. The system of any one of claims 1 to 5, wherein the processor is adapted to generate and output a PPG delay map.

7. The system of any one of claims 1 to 6, wherein the processor is adapted to determine a global PPG signal for the overall region of interest by averaging the aligned PPG signals.

8. The system of any one of claims 1 to 7, further comprising a camera for capturing the images of the region of interest.

9. The system of claim 8, wherein the camera comprises:
a 2D camera or a set of 2D cameras; or
a hyperspectral camera or a set of hyperspectral cameras.

10. A computer-implemented method for processing images to derive remote PPG signals, comprising:
(142) receiving images of a region of interest;
(144) determining PPG signals from different image regions of the images;
(146) determining relative delays between the PPG signals for the different image regions;
(148) improving the alignment between the PPG signals in time; and
(149) determining a global PPG signal for the overall region of interest by combining the time-adapted PPG signals.

11. The method of claim 10, comprising:
deriving a reference PPG signal;
determining the relative delays between the PPG signals by determining a delay of each PPG signal relative to the reference PPG signal; and
aligning the PPG signals in time by providing a common delay relative to the reference PPG signal.

12. The method of claim 11, comprising obtaining the reference PPG signal by:
averaging the PPG signals for the region of interest; or
averaging the PPG signals for a portion of the region of interest; or
obtaining a PPG signal for an individual image region.

13. The method of any one of claims 10 to 12, comprising:
deriving a PPG perfusion map based on PPG amplitude levels at the image regions; and/or
deriving a PPG delay map based on PPG relative delays between the different image regions.

14. A computer program comprising computer program code means which is adapted, when said program is run on a computer, to implement the method of any one of claims 10 to 13.

15. A processor which is programed with the computer program of claim 14.
